# EUROPÄISCHE PATENTSCHRIFT

(11) **EP 0 740 661 B1**
(45) Veröffentlichungstag und Bekanntmachung des Hinweises auf die Patenterteilung: **18.04.2001**
(21) Anmeldenummer: 95906293.6
(22) Anmeldetag: 04.01.1995
(51) Int. Cl.: C07D 263/10, A01N 43/76

(54) **DIPHENYLOXAZOLIN-DERIVATE**
DIPHENYL OXAZOLINE DERIVATIVES
DERIVES DE DIPHENYLOXAZOLINE

(30) Priorität: 17.01.1994 DE 4401098
(43) Veröffentlichungstag der Anmeldung: 06.11.1996
(73) Patentinhaber: BAYER AG, 51368 Leverkusen (DE)
(72) Erfinder: KANELLAKOPULOS, Johannes, D-41542 Dormagen (DE); KLEEFELD, Gerd, D-41468 Neuss-Üdesheim (DE); KRAATZ, Udo, D-51375 Leverkusen (DE); WACHENDORFF-NEUMANN, Ulrike, D-40789 Monheim (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: EP9500022
(87) Internationale Veröffentlichungsnummer: WO9519350

(56) Entgegenhaltungen:
- WO-A-95/04726
- CHEMICAL ABSTRACTS, Band 121, Nr. 17, veröffentlicht 1994, 24 Oktober, (Columbus, Ohio, USA), YAMADA, Y. et al. "Preparation of oxazoles and analogs as pesticides", Seite 1172, Spalte 2, Nr. 205 334a; & JP,A,06 145 155 (JPN. KOKAI TOKKYO KOHO).
- CHEMICAL ABSTRACTS, Band 117, Nr. 13, veröffentlicht 1992, 28 September, (Columbus, Ohio, USA), MIYAMOTO, S. et al. "Preparation of 2,4-diphenyl-2-oxazoline derivatives as insecticides and acaricides", Seite 745, Spalte 1, Nr. 131 181s; & JP,A,04 089 484 (JPN. KOKAI TOKKYO KOHO).
- CHEMICAL ABSTRACTS, Band 116, Nr. 25, veröffentlicht 1992, 22 Juni, (Columbus, Ohio, USA), A.I. MEYERS et al. "A highly stereoselective syntesis of axially chiral biaryls. Application to the synthesis of a potential chiral catalyst", Seite 752, Spalte 1, Nr. 255 251p; & Tetrahedron Lett. 1992, 33(7), 853-6 (Eng.).

## Beschreibung

Die Erfindung betrifft neue Diphenyloxazolin-Derivate, mehrere Verfahren zu ihrer Herstellung und ihre Verwendung zur Bekämpfung von tierischen Schädlingen.

Es ist bereits bekannt, daß bestimmte Oxazolin-Derivate insektizide und akarizide Eigenschaften aufweisen (vgl. z.B. EP-A 0 345 775 und EP-A 0 432 661). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen Diphenyloxazolin-Derivate der Formel (I) gefunden, 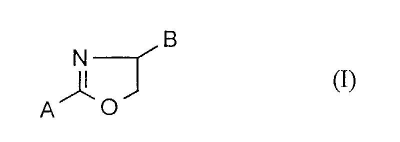 in welcher
- A: für Phenyl steht, das dreifach bis fünffach, gleich oder verschieden substituiert ist durch
Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro oder Cyano.
- B: für Phenyl steht, das einfach bis fünffach, gleich oder verschieden substituiert ist durch
Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch weitere 1-3 Sauerstoffatome unterbrochen ist,
C₁-C₁₈-Alkylthio,
C₁-C₈-Halogenalkylthio,
gegebenenfalls durch C₁-C₄-Alkyl oder C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl oder Cyclohexyloxy;
gegebenenfalls einfach oder zweifach, gleich oder verschieden durch Halogen, C₁-C₄-Alkyl oder C₁-C₄-Halogenalkyl substituiertes Pyridyloxy;
jeweils gegebenenfalls einfach bis dreifach, gleich oder verschieden durch C₁-C₁₂-Alkyl, Halogen, Cyano, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio, C₁-C₆-Halogenalkylthio oder Trimethylsilyl substituiertes Phenyl, Benzyl, Phenethyl, Phenoxy, Phenylthio, Benzyloxy, Phenethyloxy, Benzylthio oder Styryl.

Weiterhin wurde gefunden, daß man Diphenyloxazolin-Derivate der Formel (I) erhält, wenn man
α) Aminoalkohole der Formel (II) 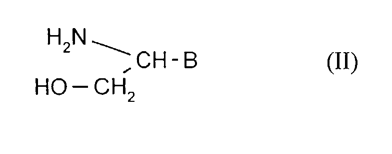 in welcher
   - B: die oben angegebene Bedeutung hat,
   mit einer Carbonsäure der Formel (III)

   A-COOH (III)

   in welcher
   - A: die oben angegebene Bedeutung hat,
   mit einem wasser-entziehenden Mittel, gegebenenfalls in Gegenwart eines Verdünnungsmittels umsetzt;
   oder
β) Amidalkohole der Formel (IV) 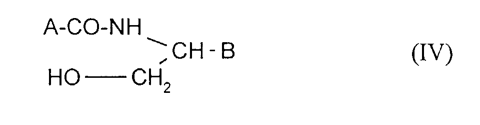 in welcher
   - A und B: die oben angegebene Bedeutung haben,
   mit einem wasser-entziehenden Mittel, gegebenenfalls in Gegenwart eines Verdünnungsmittels umsetzt;
   oder
γ) Amid-Derivate der Formel (V) 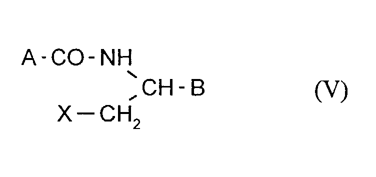 in welcher
   - A und B: die oben angegebene Bedeutung haben; und
   - X: für eine Abgangsgruppe, wie Halogen, Alkylsulfonyloxy oder Arylsulfonyloxy steht,
   mit einer Base, gegebenenfalls in Gegenwart eines Verdünnungsmittels umsetzt.

Weiterhin wurde gefunden, daß Diphenyloxazolin-Derivate der Formel (I) sehr gut zur Bekämpfung von tierischen Schädlingen geeignet sind. Sie zeichnen sich insbesondere durch hohe Wirksamkeit gegen Arthropoden und Nematoden aus.

Überaschenderweise zeigen die erfindungsgemäßen Diphenyloxazolin-Derivate der Formel (I) eine erheblich bessere Wirksamkeit gegenüber tierischen Schädlingen als die konstitutionell ähnlichsten vorbekannten Verbindungen.

Die erfindungsgemäßen Verbindungen sind durch die Formel (I) allgemein definiert.
- A: steht bevorzugt für Phenyl, das dreifach bis fünffach, gleich oder verschieden substituiert ist durch
F, Cl, Br, C₁-C₃-Alkyl, C₁-C₃-Alkoxy, C₁-C₃-Alkylthio, einfach bis fünffach, gleich oder verschieden durch F und/oder Cl substituiertes C₁-C₂-Alkyl, einfach bis fünffach, gleich oder verschieden durch F und/oder Cl substituiertes C₁-C₄-Alkoxy, SCF₃, SCHF₂, Nitro oder Cyano.
- B: steht bevorzugt für Phenyl, das einfach bis fünffach, gleich oder verschieden substituiert ist durch
F, Cl, Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
die Gruppierungen 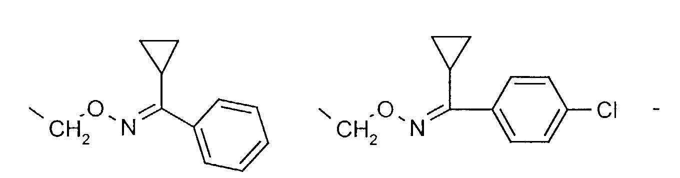 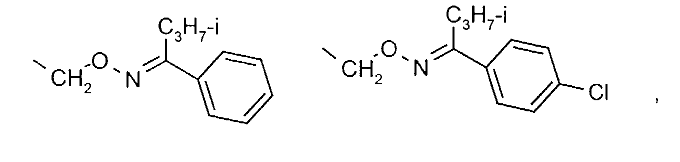 jeweils gegebenenfalls durch C₁-C₄-Alkyl, C₁-C₄-Alkoxy, Cyclohexyl oder Phenyl substituiertes Cyclohexyl oder Cyclohexyloxy;
gegebenenfalls einfach oder zweifach, gleich oder verschieden durch F, Cl oder CF₃ substituiertes Pyridyloxy;
jeweils gegebenenfalls einfach bis dreifach, gleich oder verschieden durch C₁-C₁₂-Alkyl, F, Cl, Br, Cyano, CF₃, C₁-C₄-Alkoxy, einfach bis sechsfach, gleich oder verschieden durch F und/oder Cl substituiertes C₁-C₄-Alkoxy, C₁-C4-Alkoxy-C₁-C₄-alkyl, C₁-C₄-Alkoxyethylenoxy, C₁-C₄-Alkylthio, einfach bis sechsfach, gleich oder verschieden durch F und/oder Cl substituiertes C₁-C₄-Alkylthio oder Trimethylsilyl substituiertes Phenyl, Benzyl, Phenethyl, Phenoxy, Phenylthio, Phenethyloxy, Benzyloxy, Benzylthio oder Styryl;

Die oben bei der Definition der erfindungsgemäßen Verbindungen genannten Kohlenwasserstoffreste, wie Alkyl sind - auch in Verbindung mit Heteroatomen wie Alkoxy - soweit möglich jeweils geradkettig oder verzweigt.

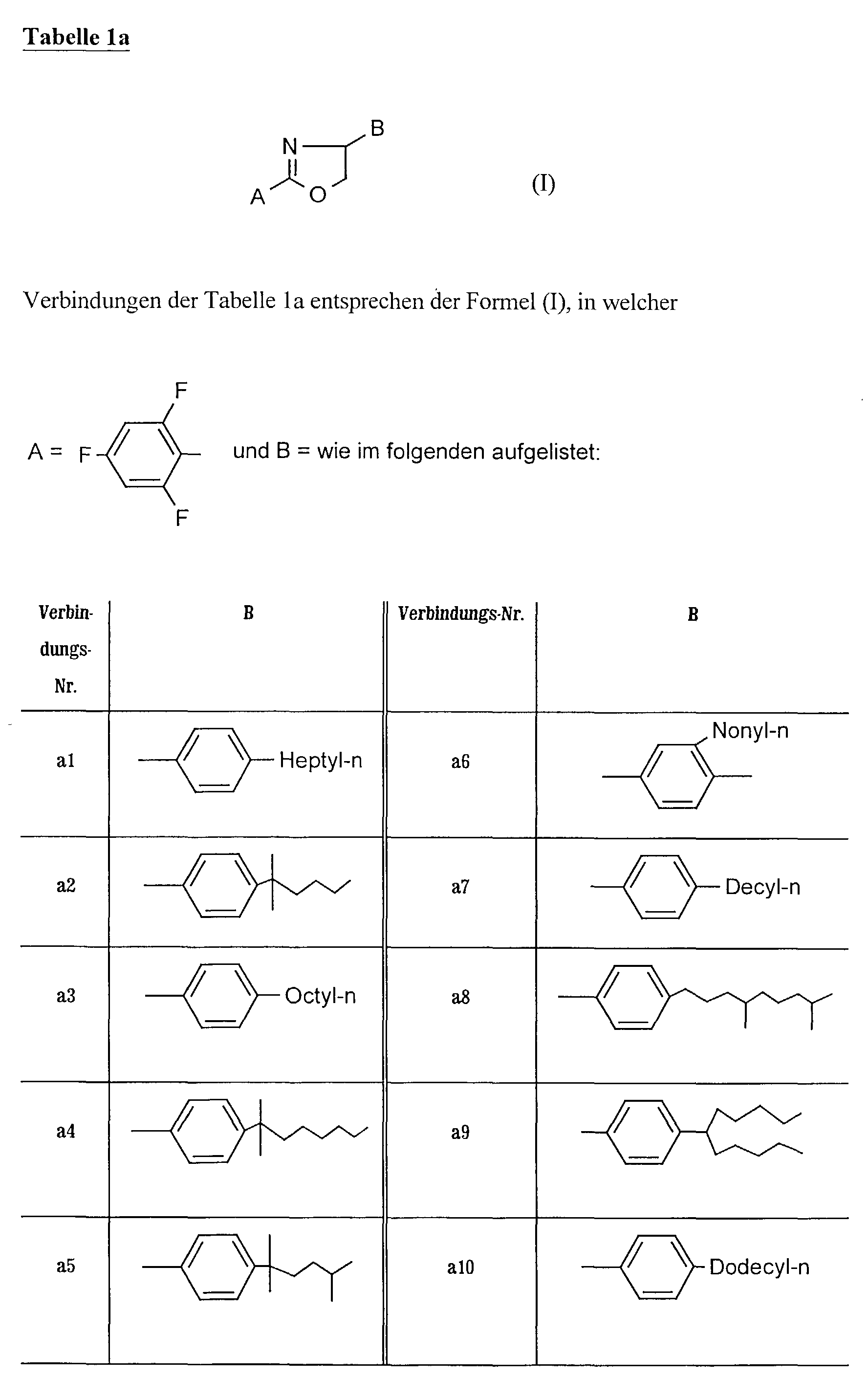

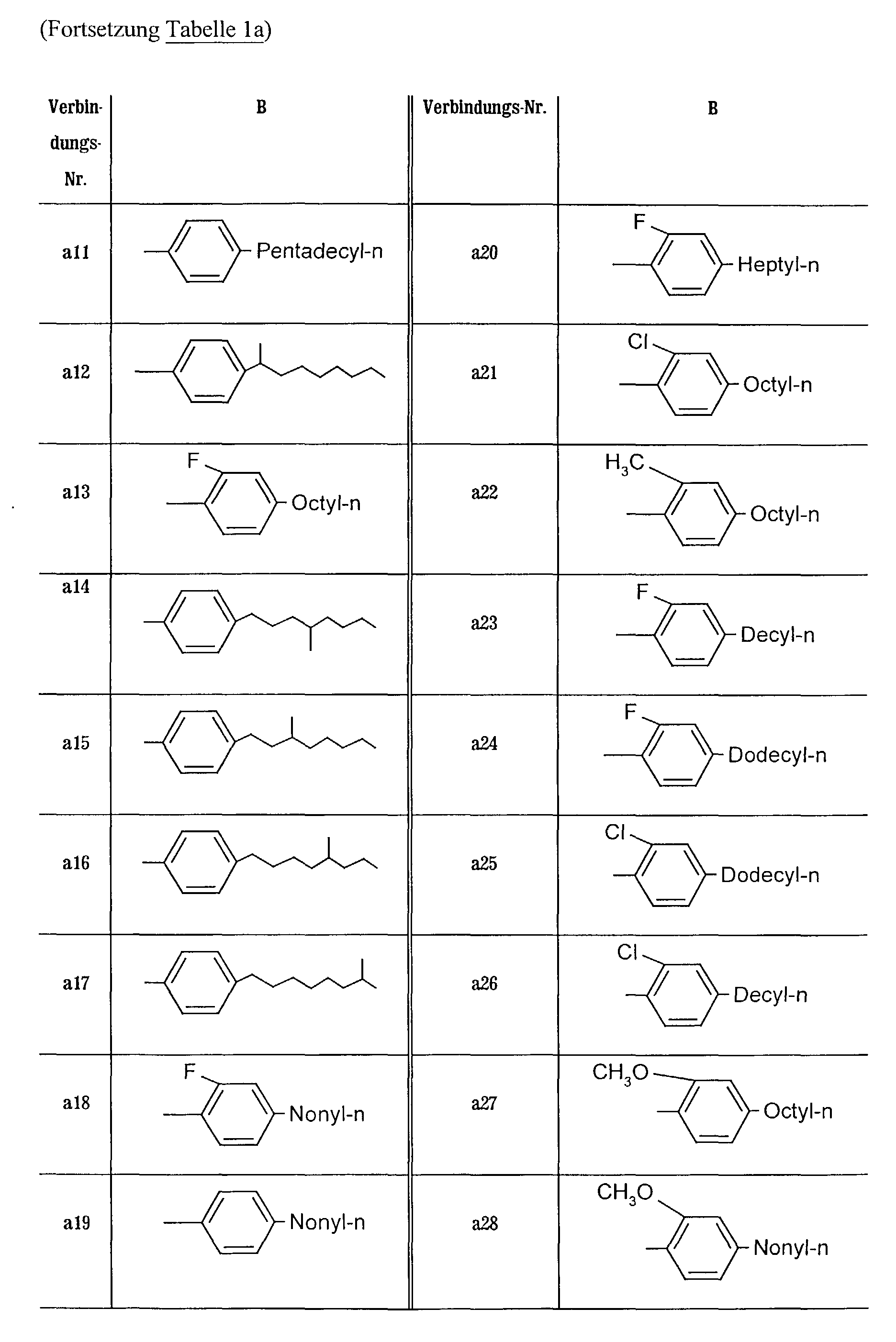

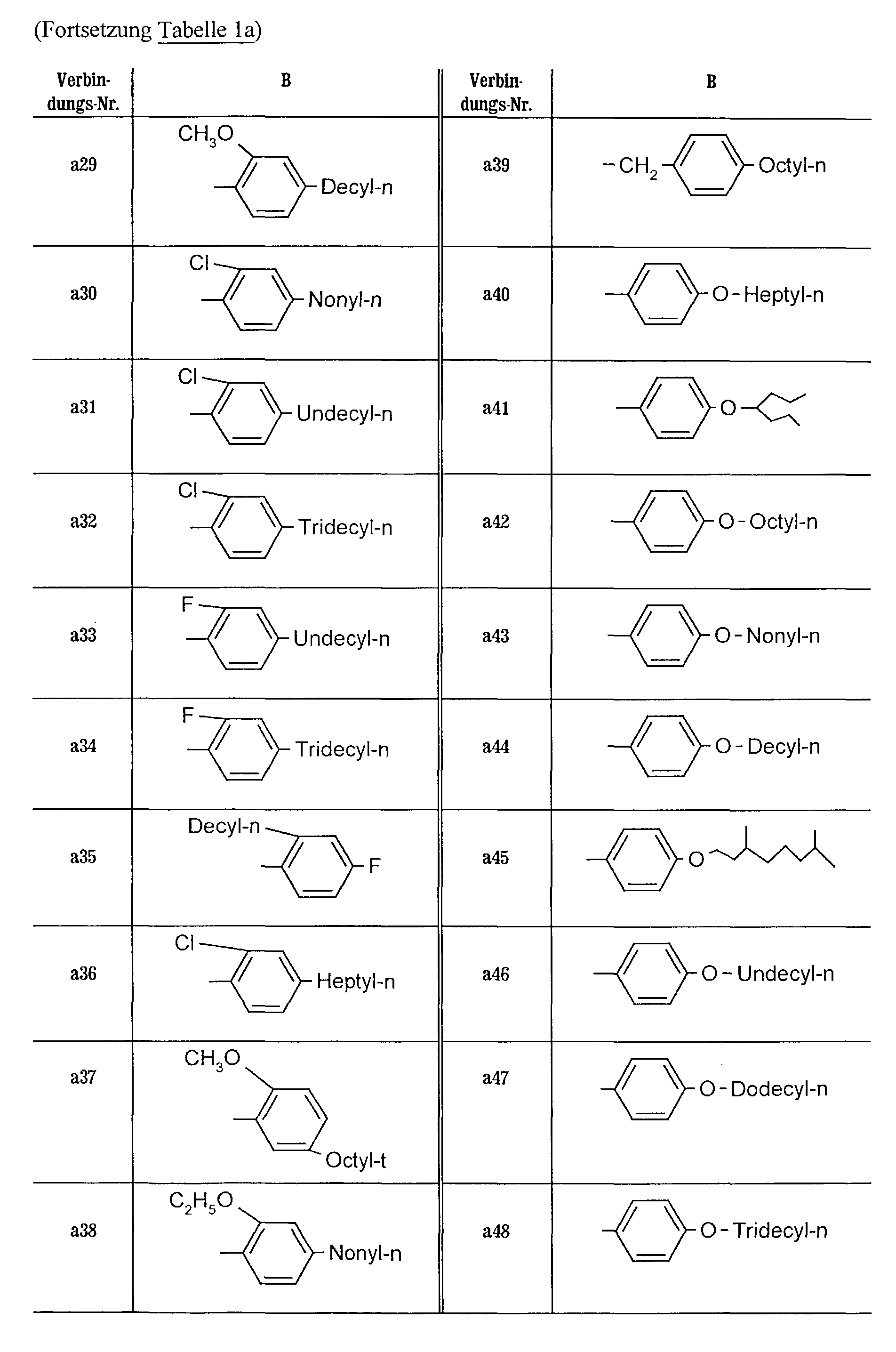

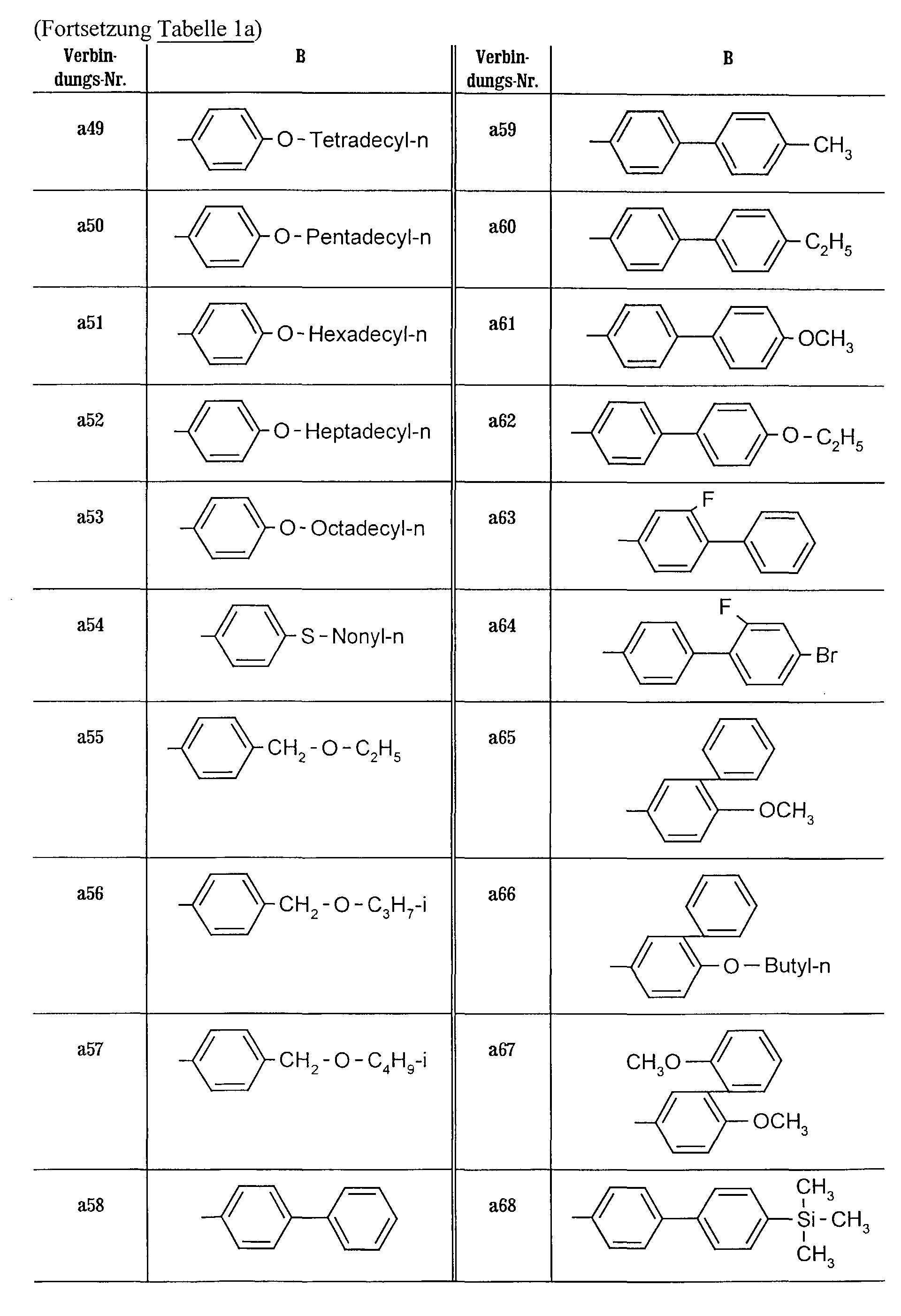

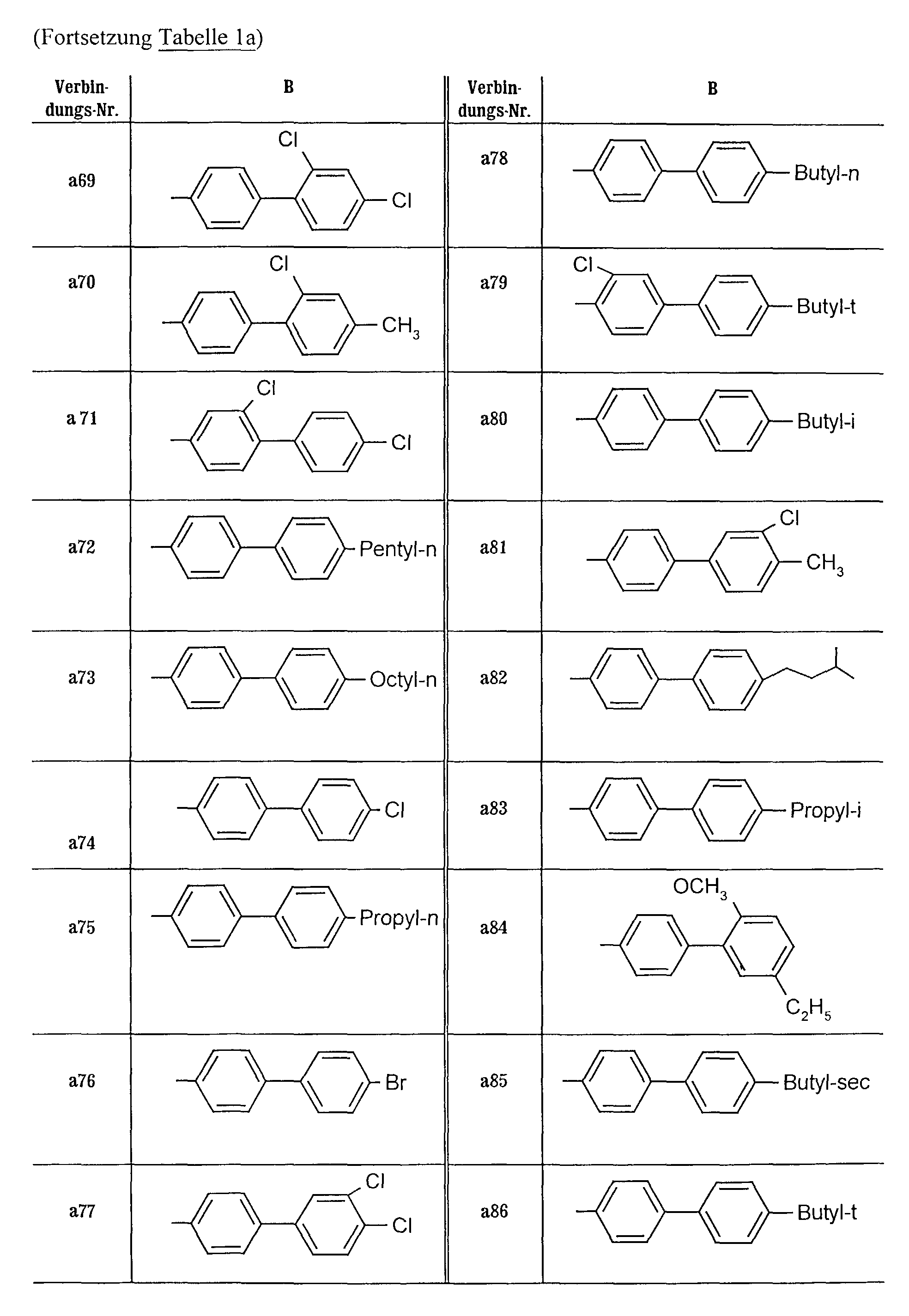

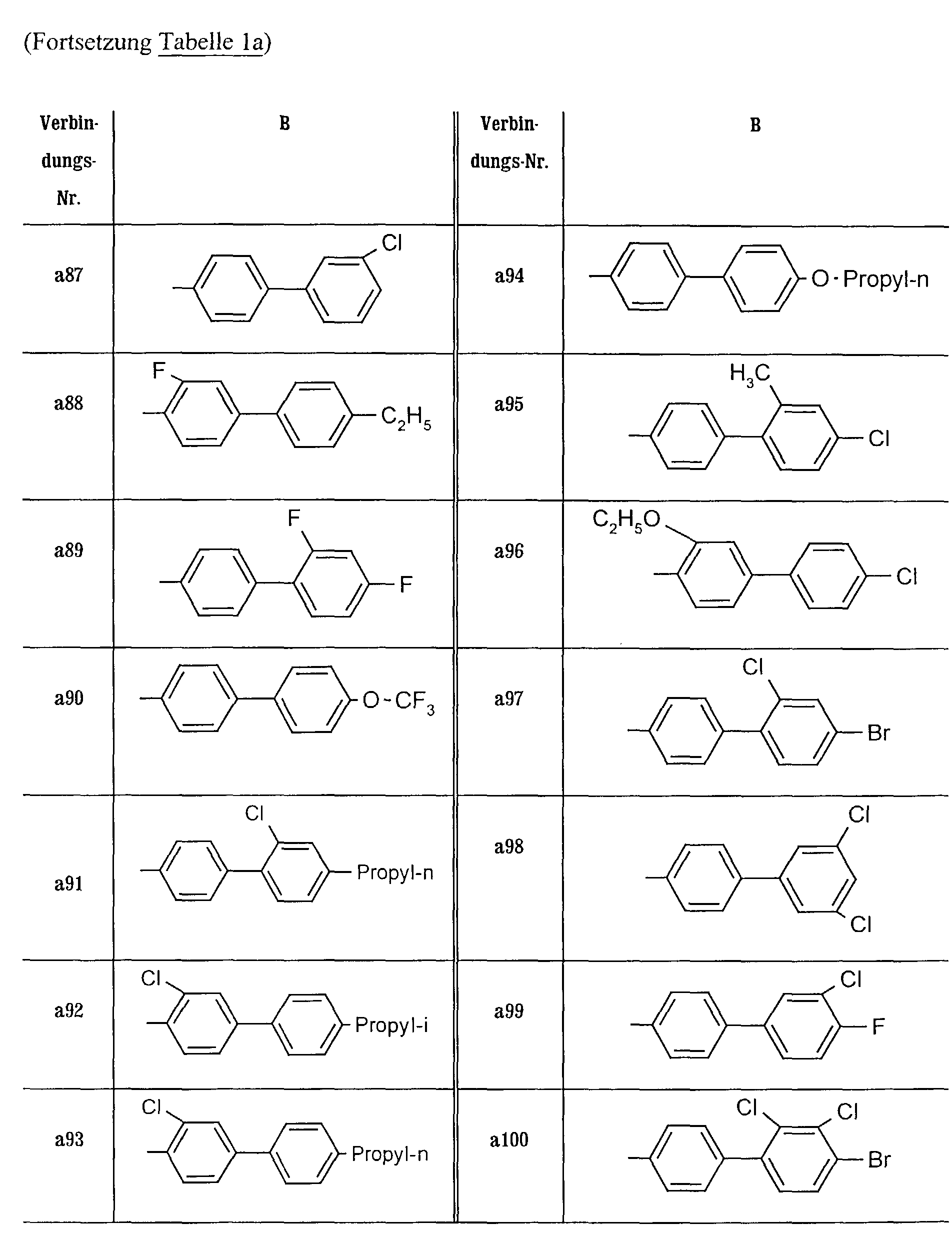

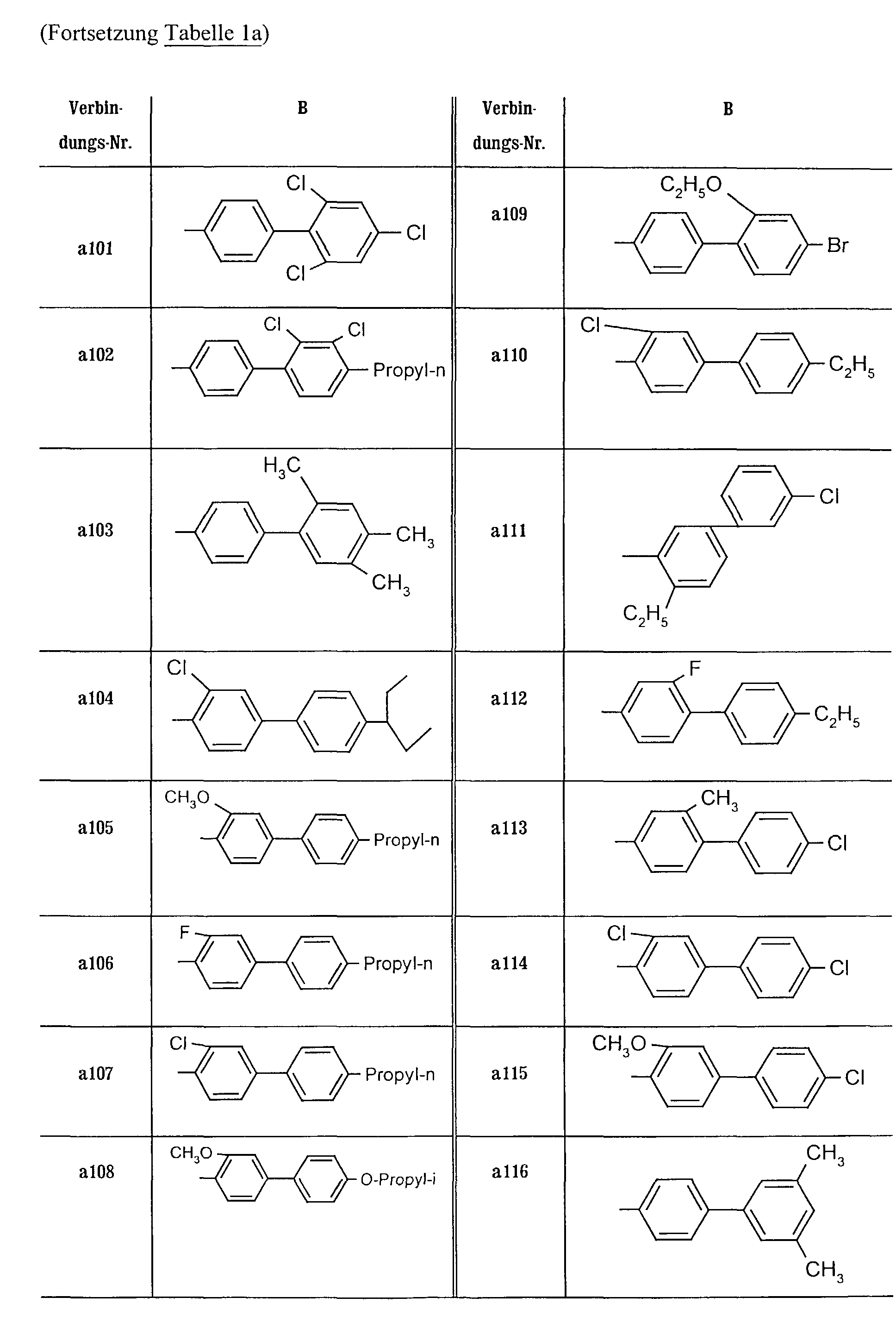

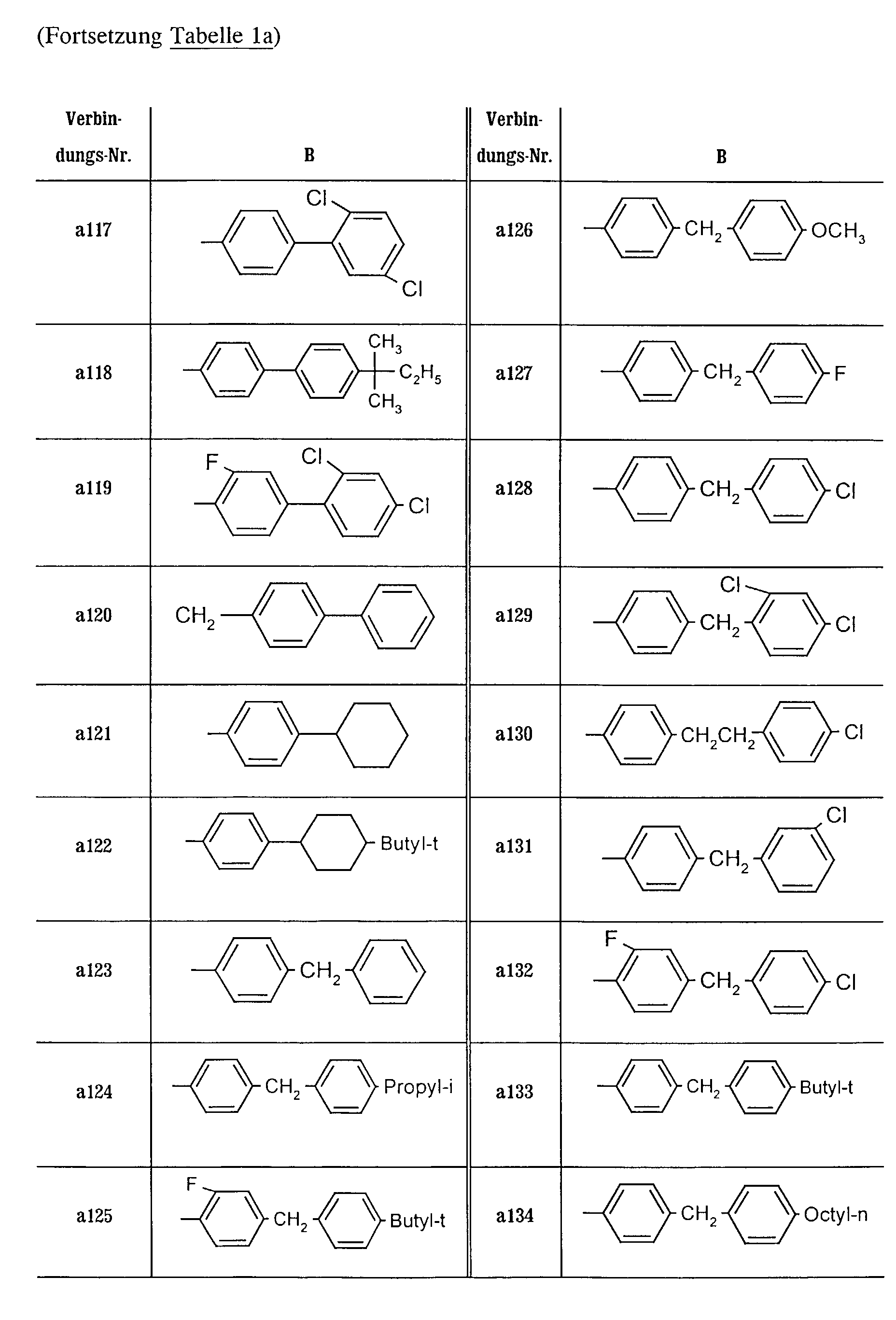

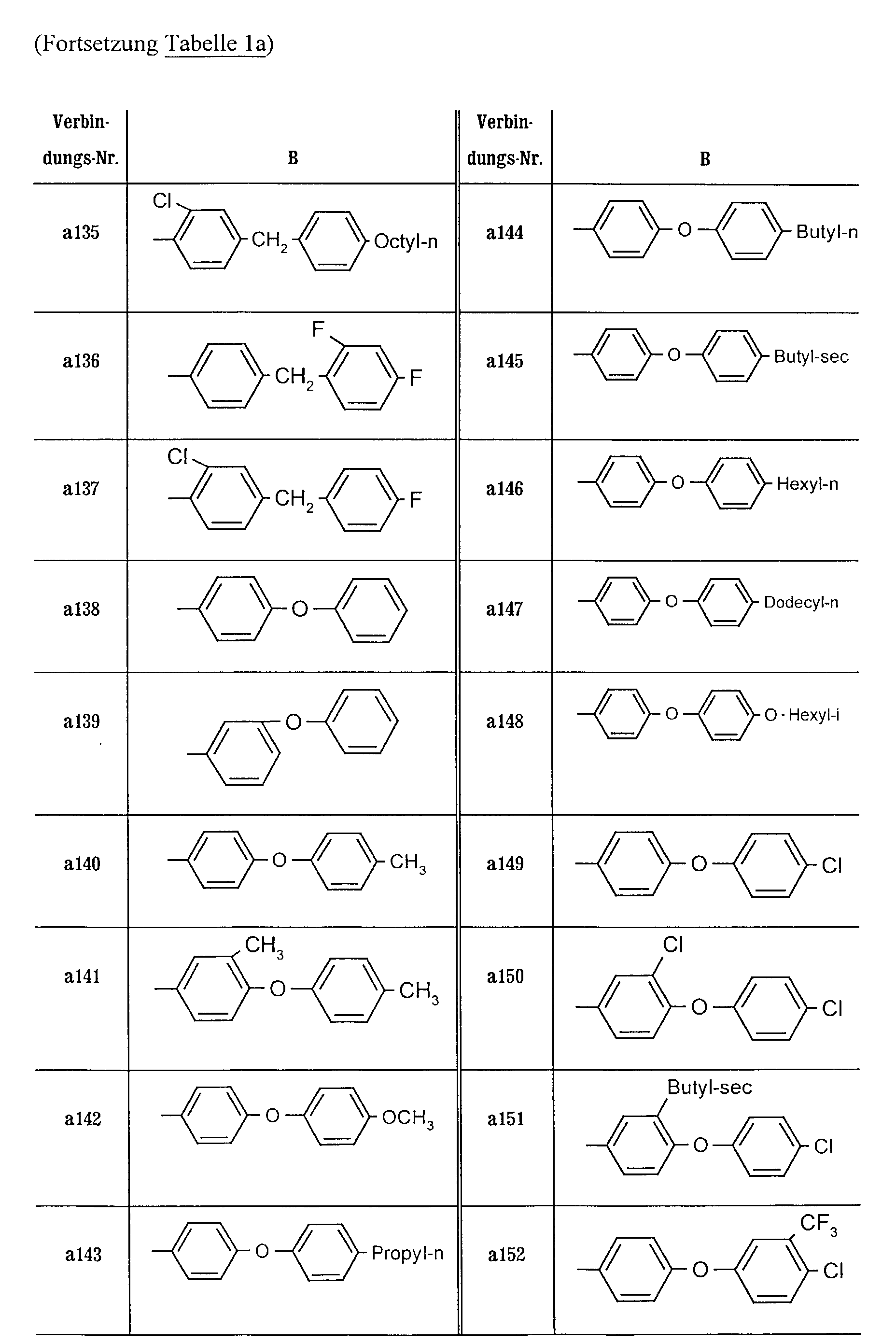

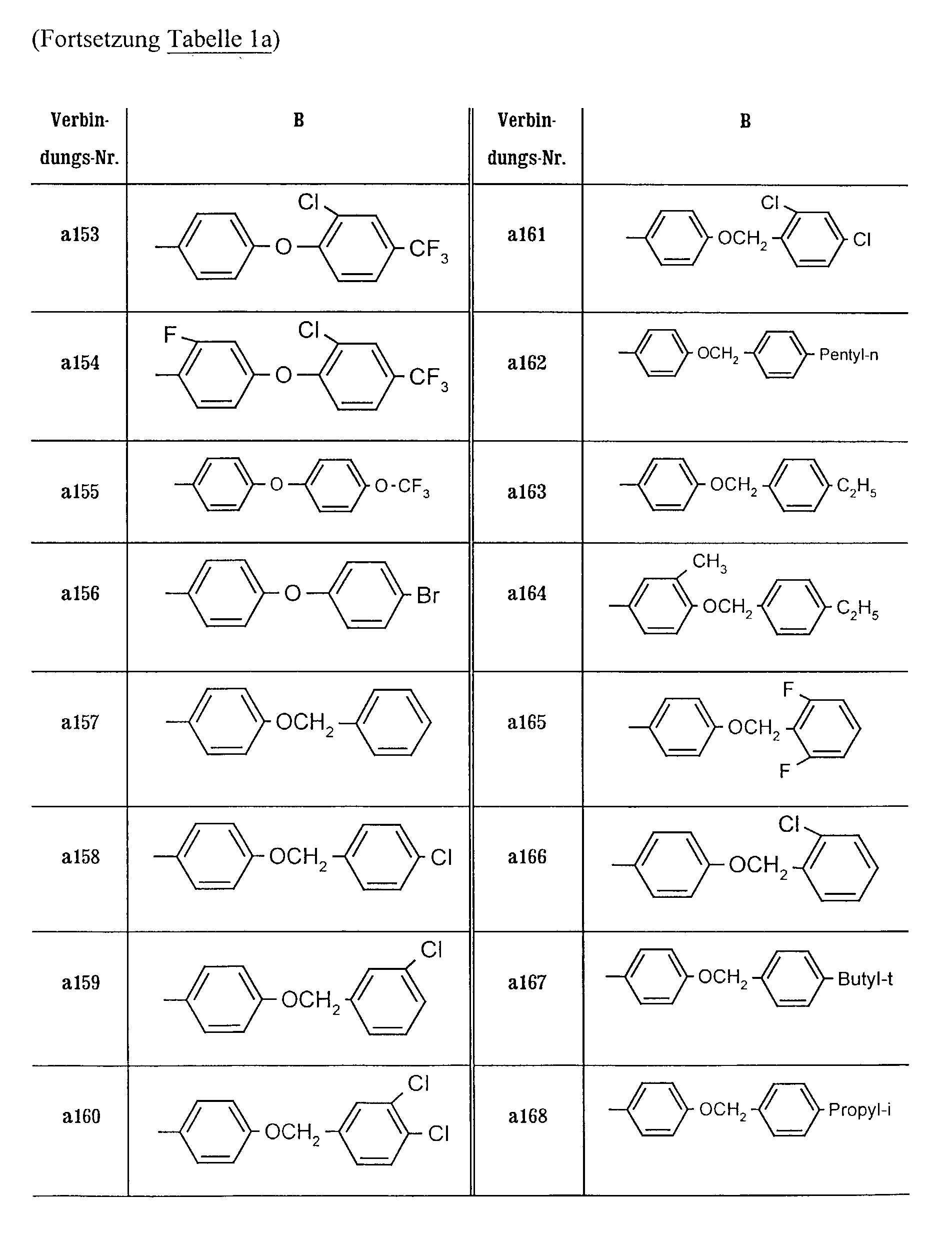

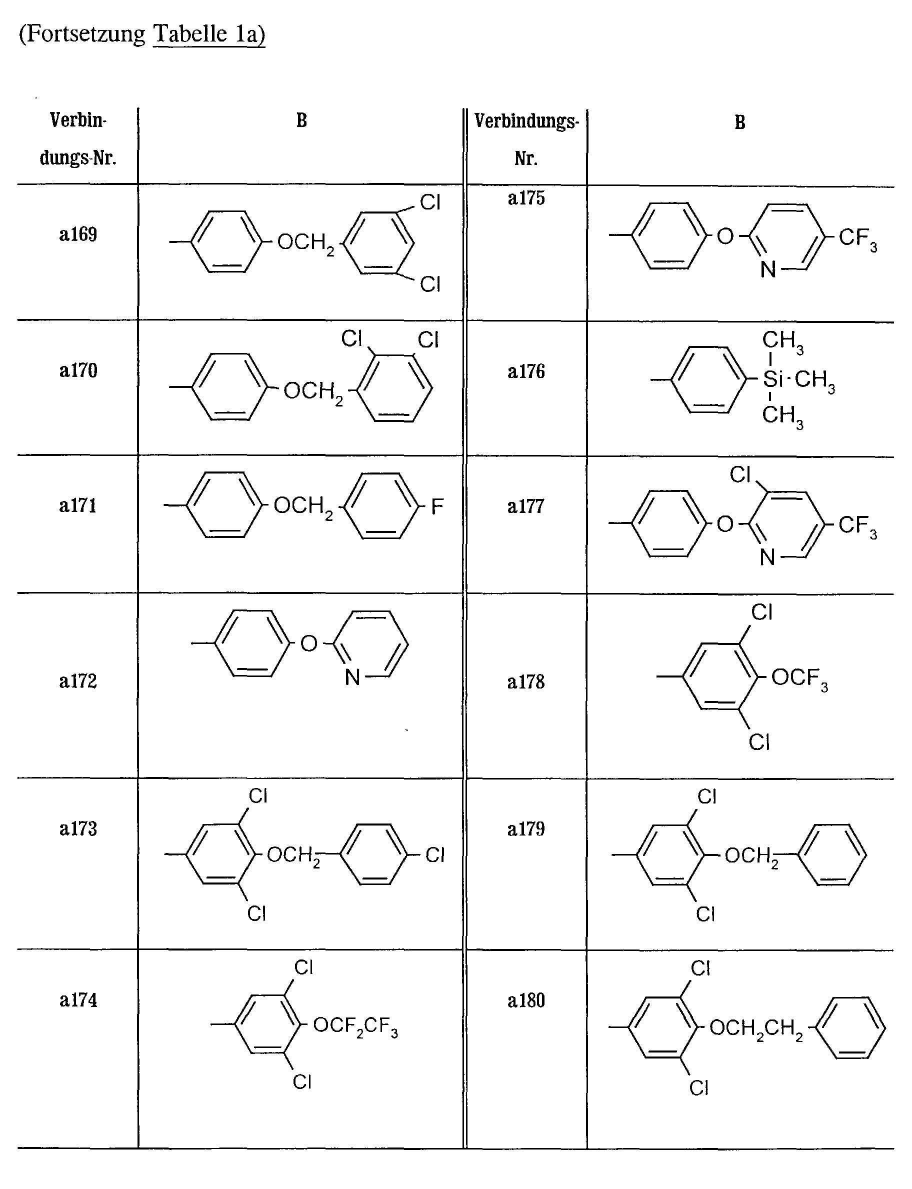

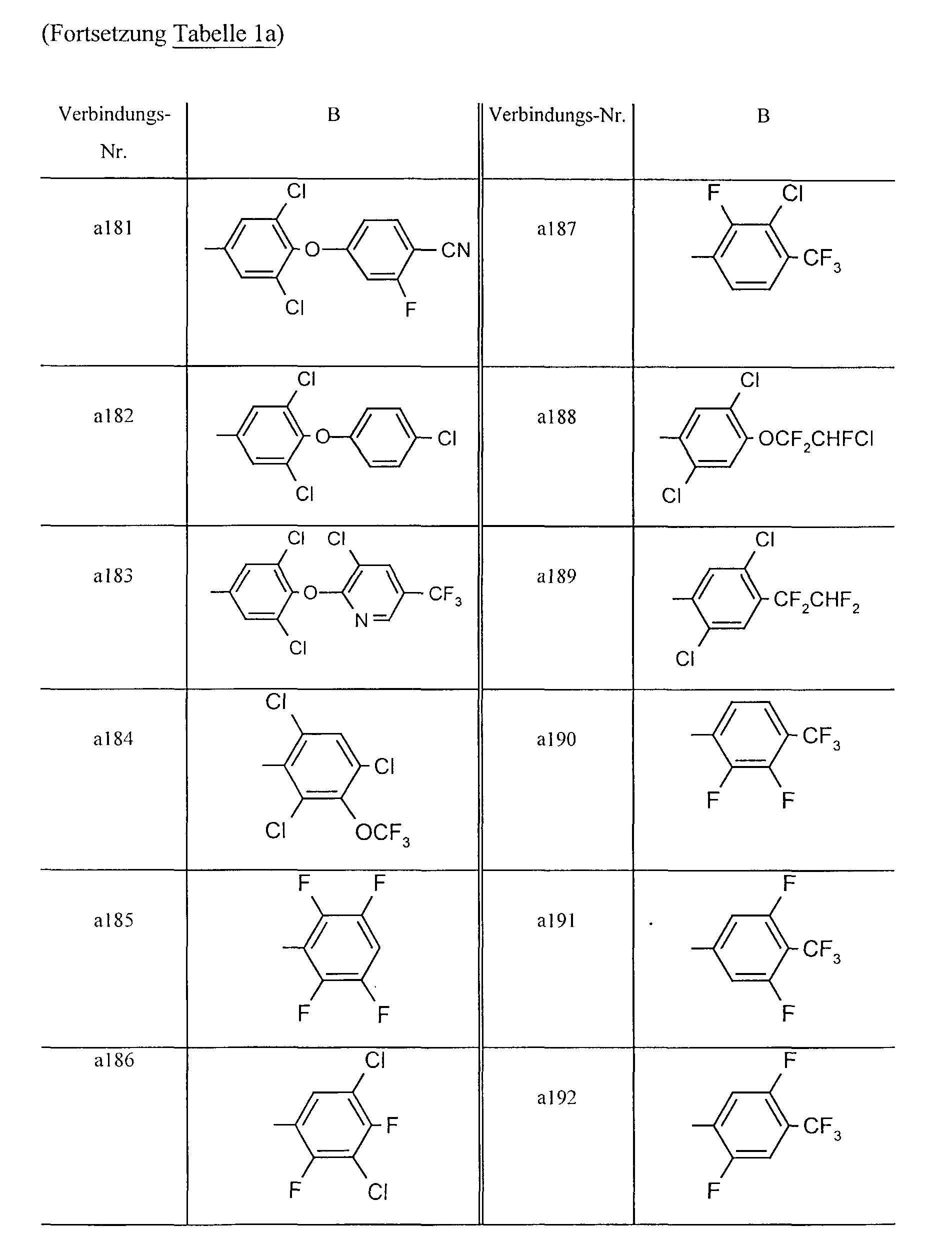

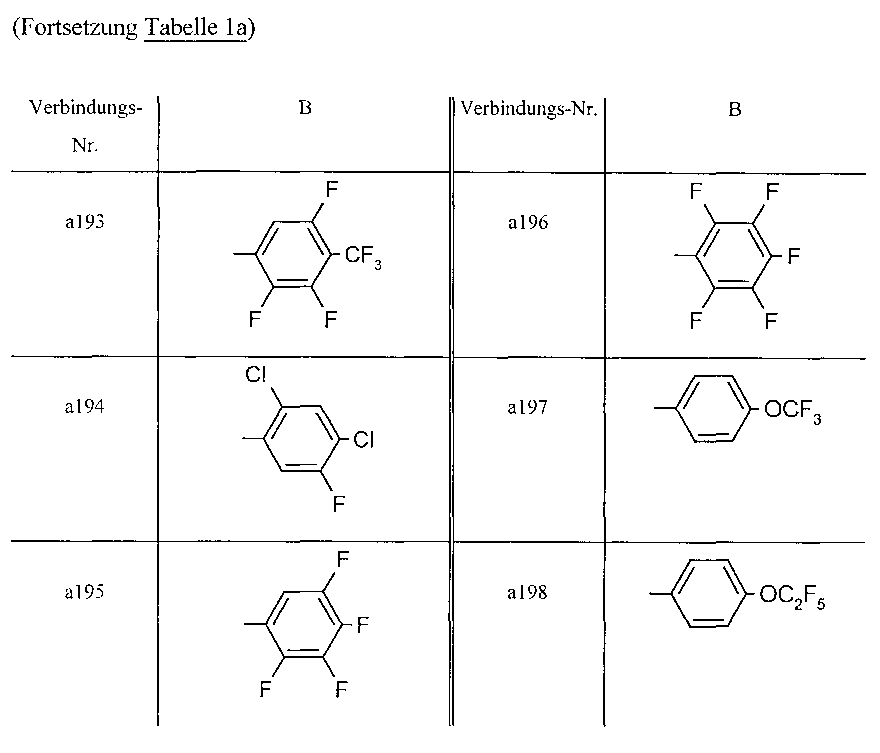

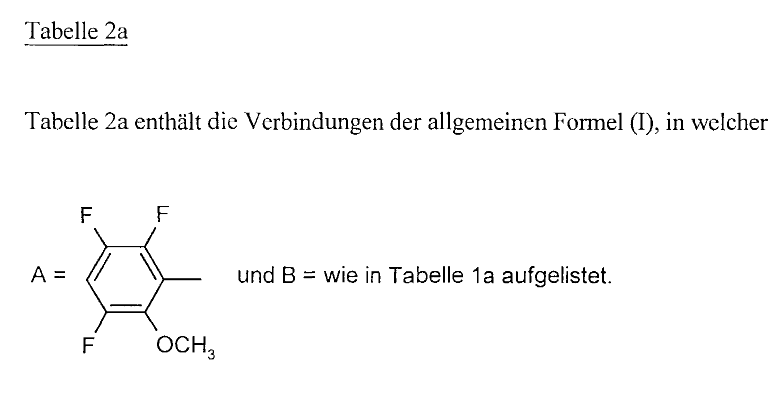

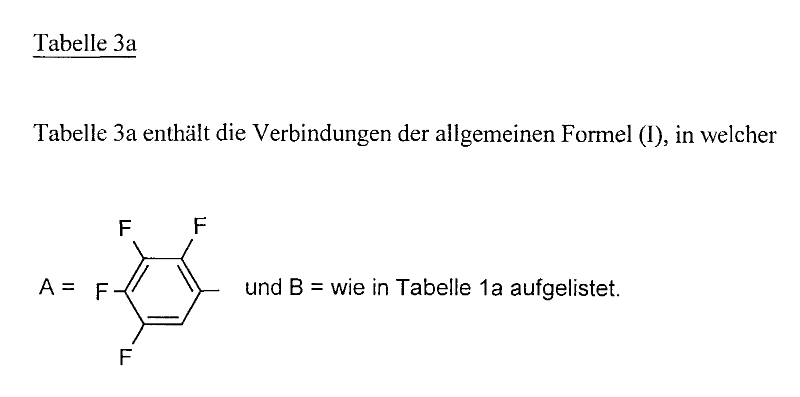

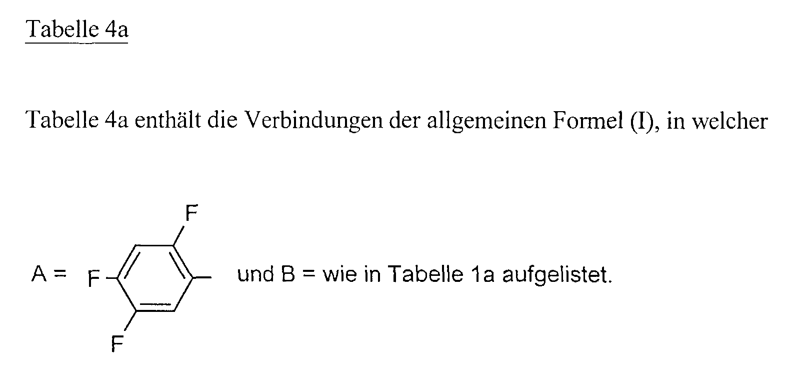

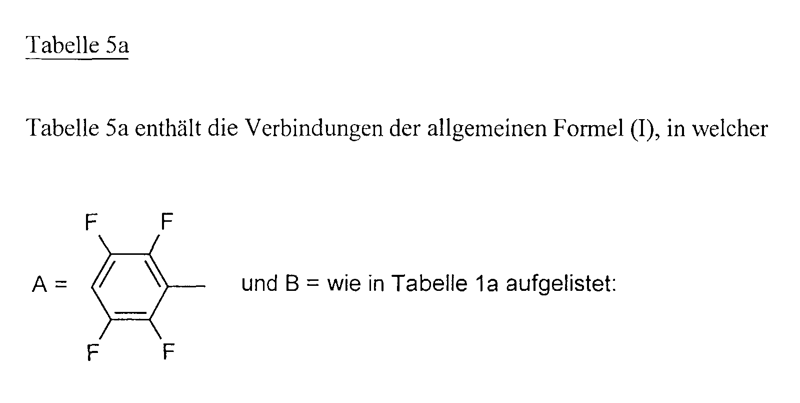

Verwendet man zur Durchführung des erfindungsgemäßen Verfahrens (α) beispielsweise 2-Amino-2-(4-t-butyl-phenyl)-1-ethanol und 2-Methoxy-3,5,6-trifluor-benzoesäure als Ausgangsstoffe und Polyphosphorsäure (PPS) als Wasser entziehendes Mittel, so kann der Reaktionsablauf durch das folgende Formelschema skizziert werden:

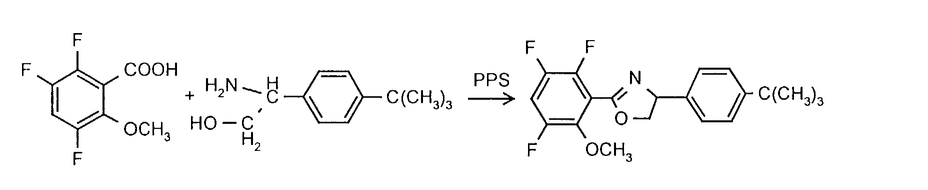

Verwendet man zur Durchführung des erfindungsgemäßen Verfahrens (β) beispielsweise N-[2-Hydroxy-1-(4-t-butyl-phenyl)-ethyl]-2-methoxy-3,5,6-trifluorbenzamid als Ausgangsverbindung und Polyphosphorsäure (PPS) als wasserentziehendes Mittel, so kann der Reaktionsablauf durch das folgende Formelschema skizziert werden:

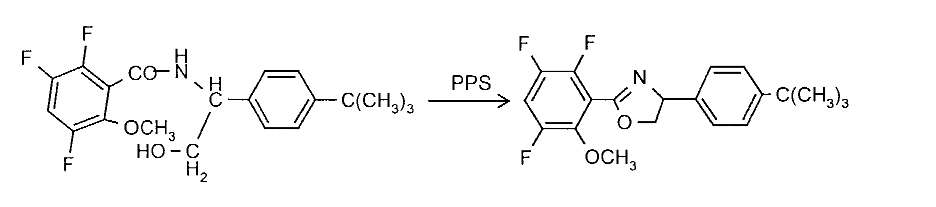

Verwendet man zur Durchführung des erfindungsgemäßen Verfahrens (γ) beispielsweise N-[2-Chlor-1-(4-t-butyl-phenyl)-ethyl]-2-methoxy-3,5,6-trifluor-benzamid als Ausgangsverbindung und Triethylamin als Base, so kann der Reaktionsablauf durch das folgende Formelschema skizziert werden:

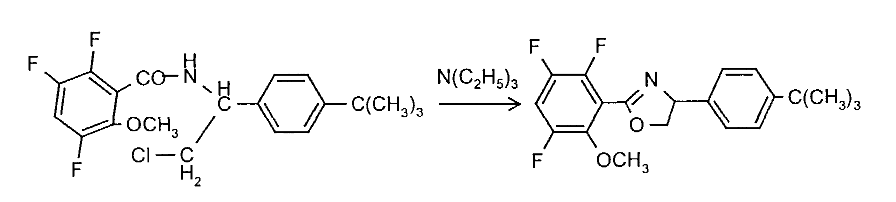

Die beim erfindungsgemäßen Verfahren (α) zur Herstellung der Verbindungen der Formel (I) als Ausgangsstoffe zu verwendenden Aminoalkohole sind durch die Formel (II) allgemein definiert. In der Formel (II) hat B vorzugsweise bzw. insbesondere diejenige Bedeutung, die bereits oben im Zusammenhang mit der Beschreibung der Verbindungen der Formel (I) als bevorzugt bzw. als insbesondere bevorzugt für B angegeben wurde.

Die Ausgangsstoffe der Formel (II) sind bekannt und/oder können nach an sich bekannten Verfahren durch Reduktion der entsprechenden Aminosäuren hergestellt werden.

Die beim erfindungsgemäßen Verfahren (α) zur Herstellung der Verbindungen der Formel (I) weiter als Ausgangsstoffe zu verwendenden Carbonsäuren sind durch die Formel (III) allgemein definiert. In der Formel (III) hat A vorzugsweise bzw. insbesondere diejenige Bedeutung, die bereits oben im Zusammenhang mit der Beschreibung der Verbindungen der Formel (I) als bevorzugt bzw. als insbesondere bevorzugt für A angegeben wurde.

Die Ausgangsstoffe der Formel (III) sind bekannte organischen Synthesechemikalien.

Die erfindungsgemäßen Verfahren (α) und (β) werden unter Verwendung eines wasserentziehenden Mittels durchgeführt. Es können die in der organischen Chemie üblichen wasserentziehenden Mitttel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid und das System Triphenylphosphin/Triethylamin/Tetrachlormethan.

Als Verdünnungsmittel zur Durchführung der erfindungsgemäßen Verfahren (α) bis (γ) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methylisobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol oder Ethanol.

Die Reaktionstemperaturen können bei der Durchführung des erfindungsgemäßen Verfahrens (α) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (α) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (α) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasserentziehenden Mittels durchgeführt, und das Reaktionsgemisch wird mehrere Stunden bei der jeweils erforderlichen Temperatur gerührt. Die Aufarbeitung erfolgt nach üblichen Methoden.

In einer besonderen Ausführungsform des erfindungsgemäßen Verfahrens (α) können statt der Carbonsäuren der Formel (III) auch entsprechende Nitrile eingesetzt werden, wobei dann vorzugsweise an Stelle eines wasserentziehenden Mittels ein Katalysator, wie z.B. Zink-(II)-Chlorid verwendet wird.

Die bei dem erfindungsgemäßen Verfahren (β) zur Herstellung der Verbindungen der Formel (I) als Ausgangsstoffe zu verwendenden Amidalkohole sind durch die Formel (IV) allgemein definiert. In der Formel (IV) haben A und B vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A und B angegeben wurden.

Die Ausgangsstoffe der Formel (IV) sind bekannt und/oder können nach an sich bekannten Verfahren hergestellt werden.

Man erhält die Amidalkohole der Formel (IV) beispielsweise, wenn man von den Carbonsäuren der Formel (III) abgeleitete Säurechloride mit Aminoalkoholen der Formel (II) in Gegenwart eines Säurebindemittels, wie z.B. Triethylamin, Pyridin, Kaliumcarbonat, Natriumhydroxid oder Kalium-t-butylat, und gegebenenfalls in Gegenwart eines Verdünnungsmittels, wie z.B. Toluol, Chlorbenzol, Aceton oder Acetonitril, bei Temperaturen zwischen 0°C und 100°C umsetzt.

Die Reaktionstemperaturen können bei der Durchführung des erfindungsgemäßen Verfahrens (β) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (β) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (β) zur Herstellung der Verbindungen der Formel (I) setzt man pro Mol an Amidalkohol der Formel (IV) im allgemeinen 1 bis 20 Mol, vorzugsweise 1 bis 5 Mol an wasserentziehendem Mittel ein.

In einer bevorzugten Ausführungsform des erfindungsgemäßen Verfahrens (β) wird der Amidalkohol der Formel (IV) in einem Verdünnungsmittel vorgelegt und das wasserentziehende Mittel dann eindosiert. Das Reaktionsgemisch wird bei der erforderlichen Temperatur bis zum Ende der Umsetzung gerührt und anschließend auf übliche Weise aufgearbeitet.

Die beim erfindungsgemäßen Verfahren (γ) zur Herstellung der Verbindungen der Formel (I) als Ausgangsstoffe zu verwendenden Amid-Derivate sind durch die Formel (V) allgemein definiert. In der Formel (V) haben A und B vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A und B angegeben wurden; X steht vorzugsweise für Fluor, Chlor, Brom, Iod, C₁-C₄-Alkyl-sulfonyloxy, Phenylsulfonyloxy oder Tolylsulfonyloxy, insbesondere für Chlor, Brom, Methylsulfonyloxy oder Tolylsulfonyloxy.

Die Ausgangsstoffe der Formel (V) sind bekannt und/oder können nach an sich bekannten Verfahren hergestellt werden.

Man erhält die Amid-Derivate der Formel (V) beispielsweise, wenn man entsprechende Amidalkohole der Formel (IV) mit Halogenierungsmitteln, wie z.B. Thionylchlorid oder Phosphor(V)-chlorid, bzw. mit Sulfonylierungsmitteln, wie z.B. Methansulfonsäurechlorid oder p-Toluolsulfonsäurechlorid auf übliche Weise umsetzt.

Das erfindungsgemäße Verfahren (γ) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, -amide, -alkoholate, -acetate, -carbonate und -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (γ) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (γ) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (γ) zur Herstellung der Verbindungen der Formel (I) setzt man pro Mol an Amid-Derivat der Formel (V) im allgemeinen 1 bis 3 Mol, vorzugsweise 1,0 bis 1,5 Mol einer Base ein.

In einer bevorzugten Ausführungsform des erfindungsgemäßen Verfahrens (γ) werden das Amid-Derivat der Formel (V) und eine Base in einem geeigneten Verdünnungsmittel vermischt; das Gemisch wird bei der erforderlichen Temperatur bis zum Ende der Umsetzung gerührt und anschließend auf übliche Weise aufgearbeitet. Die Wirkstoffe der Formel (I) eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..
Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Verbindungen zeigen in entsprechenden Aufwandmengen auch eine fungizide Wirksamkeit.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,

Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise *Boophilus microplus*.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe wird durch die folgenden Beispiele veranschaulicht:

### Herstellungsbeispiele

### Beispiel 1

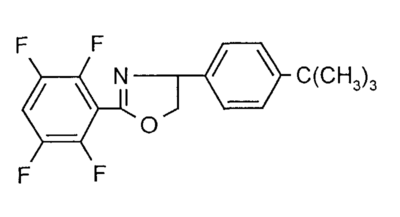

### (Verfahren γ)

Zu einer Lösung von 2,0 g (5,2 m Mol) N-[1-(4-tert.-Butylphenyl)-2-chlorethyl]-2,3,5,6-tetrafluorbenzoesäureamid in 30 ml Tetrahydrofuran gibt man 0,75 g (6,7 m Mol) Kalium-tert.-butylat und rührt bei 50°C. Nach 3 Stunden gießt man das Reaktionsgemisch auf Wasser und extrahiert mit Methylenchlorid. Die organischen Extrakte werden mit Wasser gewaschen, über Magnesiumsulfat getrocknet und anschließend im Vakuum eingeengt. Der Rückstand wird mittels Säulenchromatographie (Laufmittel Chloroform) gereinigt.

Man erhält 0,8 g (44,7% der Theorie) 4-(4-tert.-Butylphenyl)-2-(2,3,5,6-tetrafluorphenyl)-1,3-oxazolin mit einem Verteilungskoeffizienten log p (Octanol/Wasser) von 4,70 (pH: 7,4).

### Herstellung des Ausgangsproduktes

### Beisipiel (V-1)

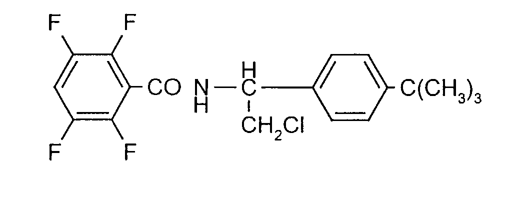

2,1 g (5,7 m Mol) N-[1-(4-tert.-Butylphenyl)-2-hydroxyethyl)-2,3,5,6-tetrafluorbenzoesäureamid werden in 50 ml Toluol mit 5 ml Thionylchlorid 18 Stunden unter Rückfluß erhitzt. Anschließend werden im Vakuum überschüssiges Thionylchlorid und das Lösungsmittel entfernt.

Man erhält 2,2 g (100% der Theorie) N-[1-(4-tert.-Butylphenyl)-2-chlorethyl]-2,3,5,6-tetrafluorbenzoesäureamid, das direkt weiter umgesetzt wird.

### Beispiel (IV-1)

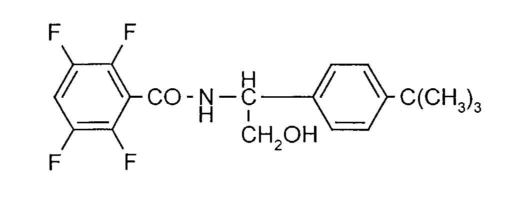

Zu einer Lösung von 1,45 g (7,5 m Mol) 2-Amino-2-(4-tert.-butylphenyl)-ethanol und 0,75 g (7,5 m Mol) Triethylamin in 20 ml Methylenchlorid tropft man unter Rühren bei 20°C 1,6 g (7,5 m Mol) 2,3,5,6-Tetrafluorbenzoylchlorid, gelöst in 10 ml Methylenchlorid, zu. Nach Sieden über Nacht verdünnt man mit Methylenchlorid, wäscht mit Wasser und engt die organische Phase im Vakuum ein. Der Rückstand wird mit wenig n-Pentan verrührt und abgesaugt.

Man erhält 2,3 g (83,1% der Theorie) N-[1-(4-tert.-Butylphenyl)-2-hydroxyethyl)-2,3,5,6-tetrafluorbenzoesäureamid vom Schmelzpunkt 158-160°C.

In entsprechender Weise und gemäß den allgemeinen Angaben zur Herstellung erhält man die folgenden Diphenyloxazolin-Derivate der Formel (I):

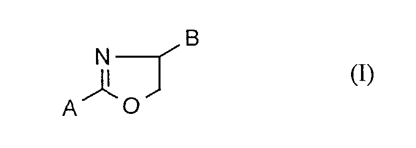

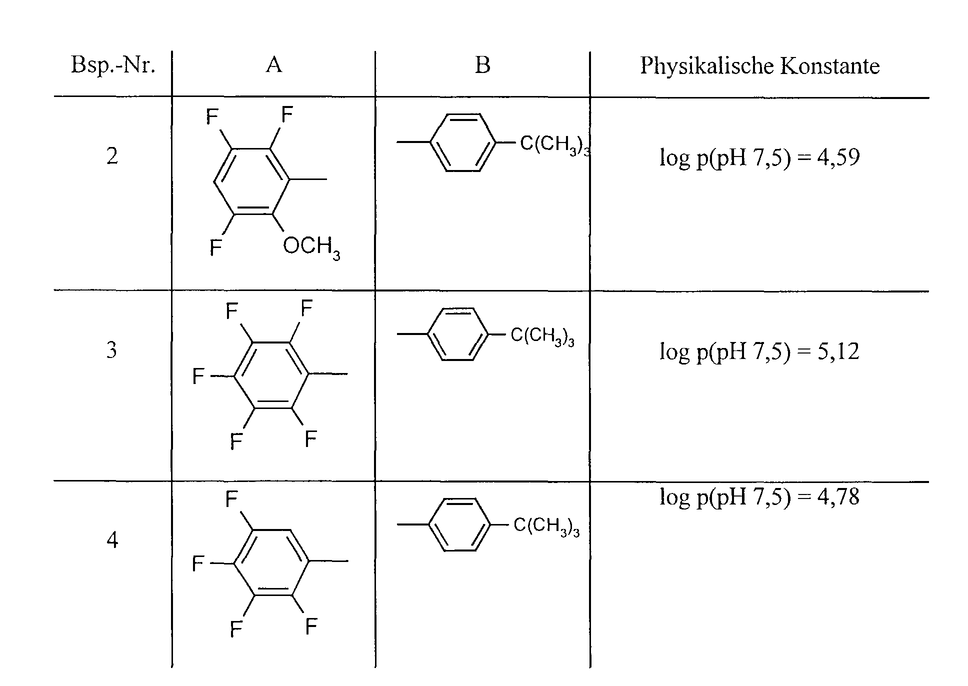

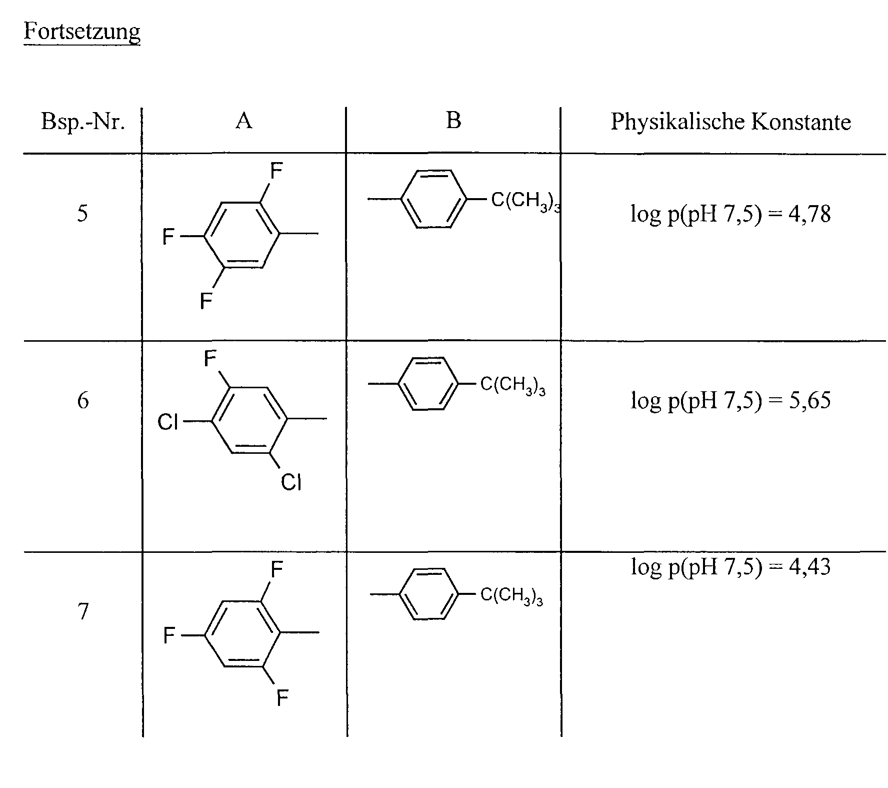

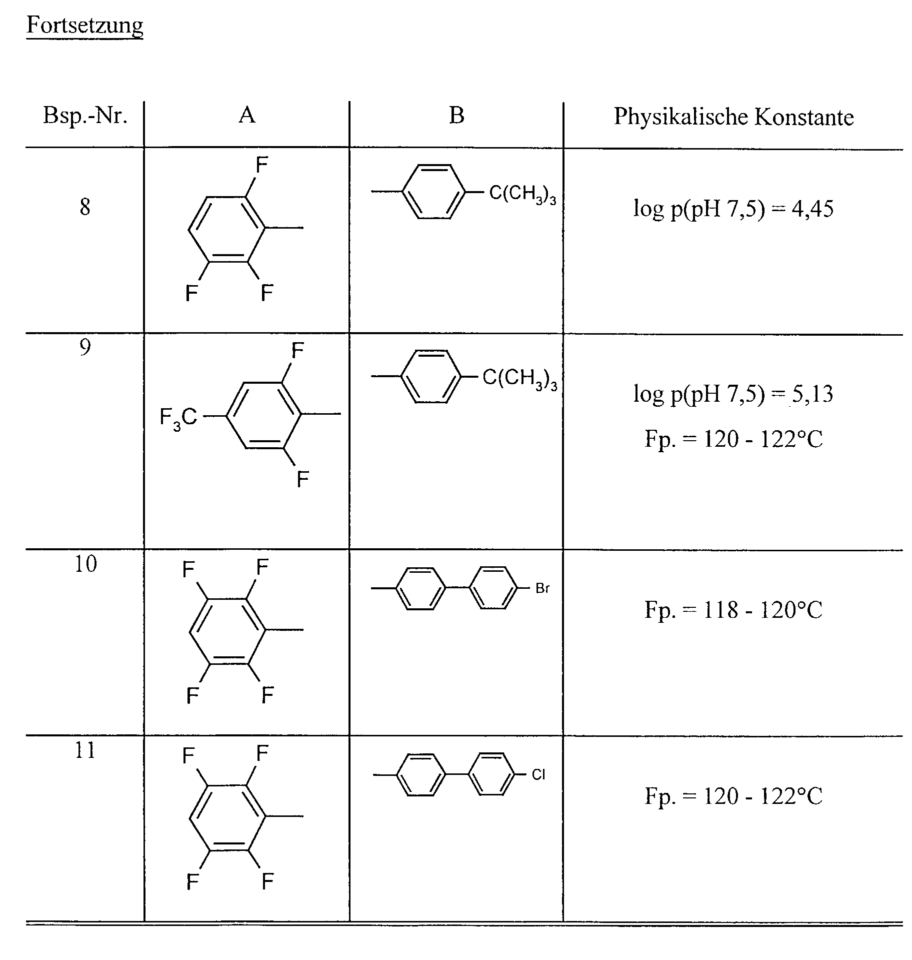

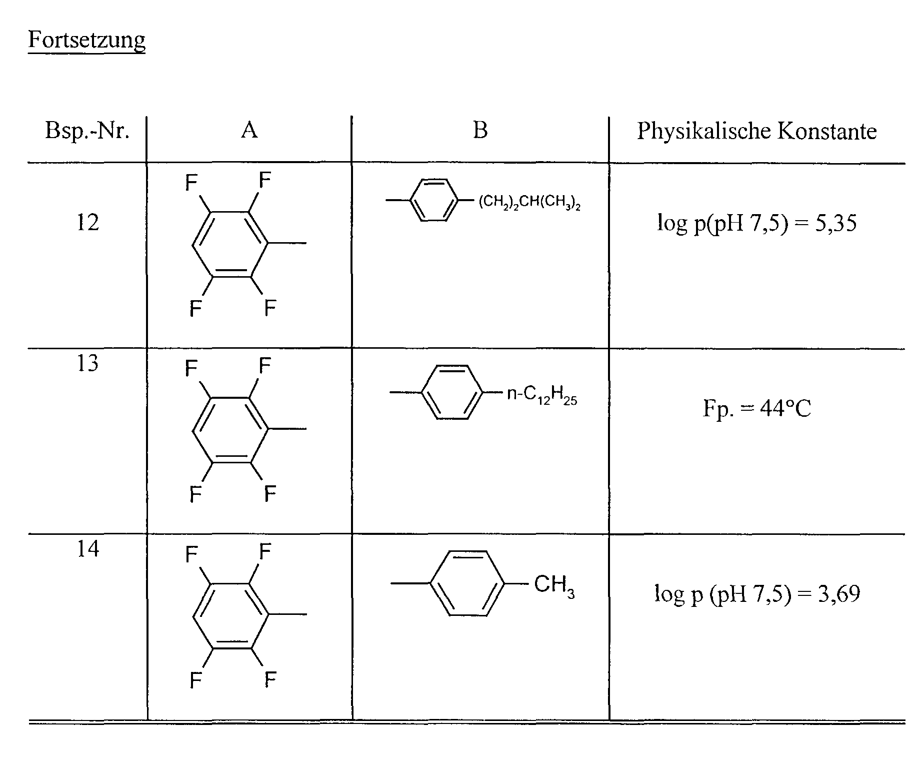

### Beispiel A

### Tetranychus-Test (OP-resistent/Tauchbehandlung)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit w-ird die Wirkung in % bestimmt. Dabei bedeutet 100%, daß alle Spinnmilben abgetötet wurden; 0% bedeutet, daß keine Spinnmilben abgetötet wurden.

Bei diesem Test zeigen z.B. die Verbindungen der Herstellungsbeispiele 1, 2 und 5 bei einer beispielhaften Wirkstoffkonzentration von 0,01% nach 7 Tagen einen Abtötungsgrad von 98%.

### Beispiel B

### Phaedon-Lanrven-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen die Verbindungen der Herstellungsbeispiele 1 und 2 bei einer Wirkstoffkonzentration von 0,1% einen Abtötungsgrad von 65%.

## Patentansprüche

1. Diphenyloxazolin-Derivate der Formel (I) 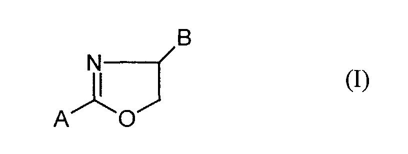 in welcher
A für Phenyl steht, das dreifach bis fünffach, gleich oder verschieden substituiert ist durch Halogen, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, Nitro oder Cyano, und
B für Phenyl steht, das einfach bis fünffach, gleich oder verschieden substituiert ist durch Halogen, C₁-C₁₈-Alkyl, C₁-C₈-Alkoxy-C₁-C₈-alkyl, C₁-C₈-Halogenalkoxy, C₁-C₄-Halogenalkyl, C₁-C₁₈-Alkoxy, das gegebenenfalls durch weitere 1-3 Sauerstoffatome unterbrochen ist, C₁-C₁₈-Alkylthio, C₁-C₈-Halogenalkylthio, gegebenenfalls durch C₁-C₄-Alkyl oder C₃-C₆-Cycloalkyl und/oder Halogen substituiertes Benzyliminooxymethyl, jeweils gegebenenfalls durch C₁-C₆-Alkyl, C₁-C₆-Alkoxy, Cyclohexyl oder Phenyl substituiertes Cyclohexyl oder Cyclohexyloxy; gegebenenfalls einfach oder zweifach, gleich oder verschieden durch Halogen, C₁-C₄-Alkyl oder C₁-C₄-Halogenalkyl substituiertes Pyridyloxy; jeweils gegebenenfalls einfach bis dreifach, gleich oder verschieden durch C₁-C₁₂-Alkyl, Halogen, Cyano, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxyethylenoxy, C₁-C₆-Alkylthio, C₁-C₆-Halogenalkylthio oder Trimethylsilyl substituiertes Phenyl, Benzyl, Phenethyl, Phenoxy, Phenylthio, Benzyloxy, Phenethyloxy, Benzylthio oder Styryl.

2. Diphenyloxazolin-Derivate der Formel (I) gemäß Anspruch 1, in welcher
A für Phenyl steht, das dreifach bis fünffach, gleich oder verschieden substituiert ist durch F, Cl, Br, C₁-C₃-Alkyl, C₁-C₃-Alkoxy, C₁-C₃-Alkylthio, einfach bis fünffach, gleich oder verschieden durch F und/oder Cl substituiertes C₁-C₂-Alkyl, einfach bis fünffach, gleich oder verschieden durch F und/oder Cl substituiertes C₁-C₄-Alkoxy, SCF₃, SCHF₂, Nitro oder Cyano, und
B für Phenyl steht, das einfach bis fünffach, gleich oder verschieden substituiert ist durch F, Cl, Br, C₁-C₁₈-Alkyl, C₁-C₆-Alkoxy-C₁-C₈-alkyl, einfach bis sechsfach, gleich oder verschieden durch F und/oder Cl substituiertes C₁-C₈-Alkoxy, einfach bis fünffach, gleich oder verschieden durch F und/oder Cl substituiertes C₁-C₂-Alkyl, C₁-C₁₈-Alkoxy und -(OC₂H₄)₁₋₃-O-C₁-C₆-alkyl, C₁-C₁₂-Alkylthio, einfach bis sechsfach, gleich oder verschieden durch F und/oder Cl substituiertes C₁-C₈-Alkylthio, die Gruppierungen 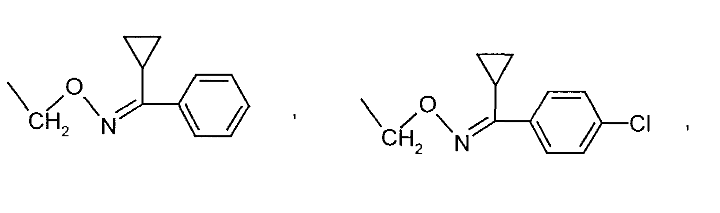 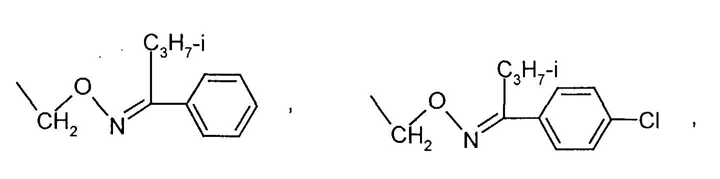 jeweils gegebenenfalls durch C₁-C₄-Alkyl, C₁-C₄-Alkoxy, Cyclohexyl oder Phenyl substituiertes Cyclohexyl oder Cyclohexyloxy; gegebenenfalls einfach oder zweifach, gleich oder verschieden durch F, Cl oder CF₃ substituiertes Pyridyloxy; jeweils gegebenenfalls einfach bis dreifach, gleich oder verschieden durch C₁-C₁₂-Alkyl, F, Cl, Br, Cyano, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxyethylenoxy, C1-C4-Alkylthio, einfach bis sechsfach, gleich oder verschieden durch F und/oder Cl substituiertes C₁-C₄-Alkylthio oder Trimethylsilyl substituiertes Phenyl, Benzyl, Phenethyl, Phenoxy, Phenylthio, Phenethyloxy, Benzyloxy, Benzylthio oder Styryl.

3. Verfahren zur Herstellung von Diphenyloxazolin-Derivaten der Formel (I) gemäß Anspruch 1 dadurch gekennzeichnet, daß man
α) Aminoalkohole der Formel (II) 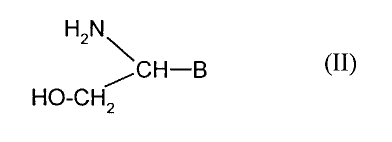 in welcher
B die oben angegebene Bedeutung hat,
mit einer Carbonsäure der Formel (III)
A-COOH (III)
in welcher
A die oben angegebene Bedeutung hat,
mit einem wasser-entziehenden Mittel und gegebenenfalls in Gegenwart eines Verdünnungsmittels umsetzt;
oder
β) Amidalkohole der Formel (IV) 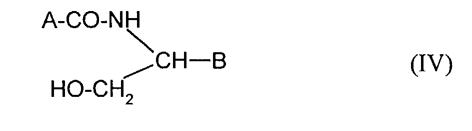 in welcher
A und B die oben angegebene Bedeutung haben,
mit einem wasser-entziehenden Mittel, gegebenenfalls in Gegenwart eines Verdünnungsmittels umsetzt;
oder
γ) Amid-Derivate der Formel (V) 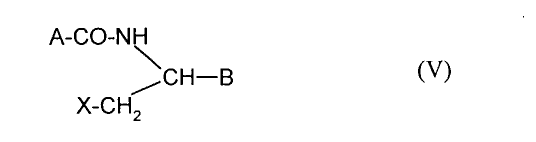 in welcher
A und B die oben angegebene Bedeutung haben; und
X für eine Abgangsgruppe steht,
mit einer Base, gegebenenfalls in Gegenwart eines Verdünnungsmittels umsetzt.

4. Schädlingsbekämpfungsmittel, gekennzeichnet durch einen Gehalt an mindestens einer Verbindung der Formel (I) gemäß Anspruch 1.

5. Verfahren zur Bekämpfung von tierischen Schädlingen,dadurch gekennzeichnet, daß man Verbindungen der Formel (I) gemäß Anspruch 1 auf tierische Schädlinge und/oder deren Lebensraum einwirken läßt.

6. Verwendung von Verbindungen der Formel (I) gemäß Anspruch 1 zur Bekämpfung von tierischen Schädlingen.

7. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

## Claims

1. Diphenyloxazoline derivatives of the formula (I) 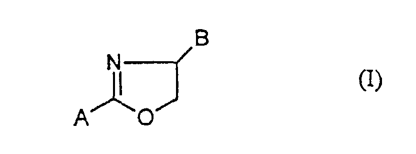 in which
A represents phenyl which is tri- to pentasubstituted by identical or different substituents consisting of halogen, C₁-C₆-alkyl, C₁-C₆-alkoxy, C₁-C₆-alkylthio, C₁-C₆-halogenoalkyl, C₁-C₆-halogenoalkoxy, C₁-C₆-halogenoalkylthio, nitro or cyano, and
B represents phenyl which is mono- to pentasubstituted by identical or different substituents consisting of halogen, C₁-C₁₈-alkyl, C₁-C₈-alkoxy-C₁-C₈-alkyl, C₁-C₈-halogenoalkoxy, C₁-C₄-halogenoalkyl, C₁-C₁₈-alkoxy which is optionally interrupted by 1-3 additional oxygen atoms, C₁-C₁₈-alkylthio, C₁-C₈-halogenoalkylthio, benzyliminooxymethyl which is optionally substituted by C₁-C₄-alkyl, or C₃-C₆-cycloalkyl and/or halogen, cyclohexyl or cyclohexyloxy each of which are optionally substituted by C₁-C₆-alkyl, C₁-C₆-alkoxy, cyclohexyl or phenyl; pyridyloxy which is optionally mono- or disubstituted by identical or different substituents consisting of halogen, C₁-C₄-alkyl or C₁-C₄-halogenoalkyl; phenyl, benzyl, phenethyl, phenoxy, phenylthio, benzyloxy, phenethyloxy, benzylthio or styryl each of which is optionally mono- to trisubstituted by identical or different substituents consisting of C₁-C₁₂-alkyl, halogen, cyano, C₁-C₄-halogenoalkyl, C₁-C₆-alkoxy, C₁-C₆-halogenoalkoxy, C₁-C₆-alkoxy-C₁-C₆-alkyl, C₁-C₆-alkoxyethyleneoxy, C₁-C₆-alkylthio, C₁-C₆-halogenoalkylthio or trimethylsilyl,

2. Diphenyloxazoline derivatives of the formula (I) according to Claim 1, in which
A represents phenyl which is tri- to pentasubstituted by identical or different substituents consisting of F, Cl, Br, C₁-C₃-alkyl, C₁-C₃-alkoxy, C₁-C₃-alkylthio, C₁-C₂-alkyl which is mono- to pentasubstituted by identical or different substituents consisting of F and/or Cl, C₁-C₄-alkoxy which is mono- to pentasubstituted by identical or different substituents consisting of F and/or Cl, or SCF₃, SCHF₂, nitro or cyano, and
B represents phenyl which is mono- to pentasubstituted by identical or different substituents consisting of F, Cl, Br, C₁-C₁₈-alkyl, C₁-C₆-alkoxy-C₁-C₈-alkyl, C₁-C₈-alkoxy which is mono- to hexasubstituted by identical or different substituents consisting of F and/or Cl, C₁-C₂-alkyl which is mono- to pentasubstituted by identical or different substituents consisting of F and/or Cl, C₁-C₁₈-alkoxy and -(OC₂H₄)₁₋₃-O-C₁-C₆-alkyl , C₁-C₁₂-alkylthio, C₁-C₈-alkylthio which is mono- to hexasubstituted by identical or different substituents consisting of F and/or Cl, the groups 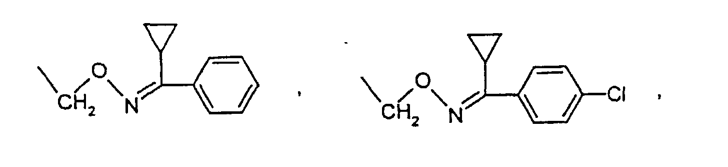 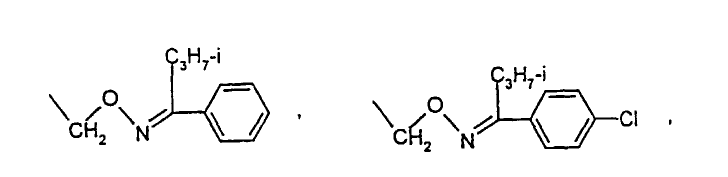 cyclohexyl or cyclohexyloxy each of which is optionally substituted by C₁-C₄-alkyl, C₁-C₄-alkoxy, cyclohexyl or phenyl; pyridyloxy which is optionally mono- or disubstituted by identical or different substituents consisting of F, Cl or CF₃; phenyl, benzyl, phenethyl, phenoxy, phenylthio, phenethyloxy, benzyloxy, benzylthio or styryl each of which is optionally mono- to trisubstituted by identical or different substituents consisting of C₁-C₁₂-alkyl, F, Cl, Br, cyano, CF3, C₁-C₄-alkoxy, C₁-C₄-alkoxy which is mono- to hexasubstituted by identical or different substituents consisting of F and/or Cl, or C₁-C₄-alkoxy-C₁-C₄-alkyl, C₁-C₄-alkoxyethyleneoxy, C₁-C₄-alkylthio, C₁-C₄-alkylthio which is mono- to hexasubstituted by identical or different substituents consisting of F and/or Cl, or trimethylsilyl

3. Process for the preparation of a diphenyloxazoline derivatives of the formula (I) according to Claim 1, characterized in that
α) amino alcohols of the formula (II) 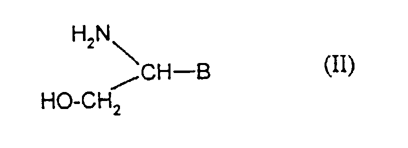 in which
B is as defined above
are reacted with a carboxylic acid of the formula (III)
A-COOH (III)
in which
A is as defined above,
with a dehydrating agent, optionally in the presence of a diluent;
or
β) amido alcohols of the formula (IV) 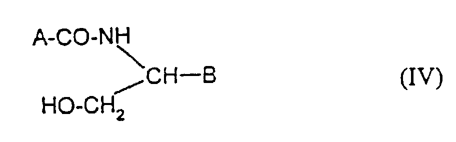 in which
A and B are as defined above
are reacted with a dehydrating agent, optionally in the presence of a diluent;
or
γ) amide derivatives of the formula (V) 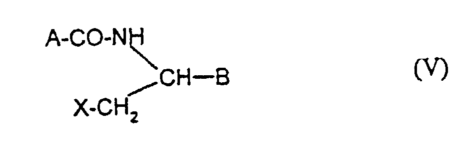 in which
A and B are as defined above and
X represents a leaving group
are reacted with a base, optionally in the presence of a diluent.

4. Pest-combating composition comprising at least one compound of the formula (I) as claimed in Claim 1.

5. Method of combating animal pests, characterized in that compounds of the formula (I) as claimed in Claim 1 are allowed to act on animal pests and/or their habitat.

6. Use of compounds of the formula (I) as claimed in Claim 1 for combating animal pests.

7. Process for the production of pest-combating compositions, characterized in that compounds of the formula (I) as claimed in Claim 1 are mixed with extenders and/or surface-active agents.

## Revendications

1. Dérivés de diphényloxazoline de formule (I) 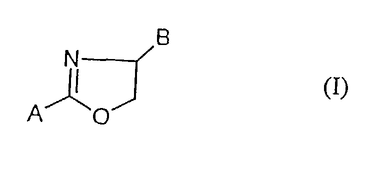 dans laquelle
A est un groupe phényle qui porte 3 à 5 substituants, identiques ou différents, halogéno, alkyle en C₁ à C₆, alkoxy en C₁ à C₆, alkylthio en C₁ à C₆, halogénalkyle en C₁ à C₆, halogénalkoxy en C₁ à C₆, halogénalkylthio en C₁ à C₆, nitro ou cyano, et
B est un groupe phényle qui porte 1 à 5 substituants, identiques ou différents, halogéno, alkyle en C₁ à C₁₈, (alkoxy en C₁ à C₈)-(alkyle en C₁ à C₈), halogénalkoxy en C₁ à C₈, halogénalkyle en C₁ à C₄, alkoxy en C₁ à C₁₈, qui est éventuellement interrompu par 1 à 3 autres atomes d'oxygène, un groupe alkylthio en C₁ à C₁₈, halogénalkylthio en C₁ à C₈, un groupe benzylimino-oxyméthyle éventuellement substitué par un radical alkyle en C₁ à C₄ ou cycloalkyle en C₃ à C₆ et/ou par un halogène, un groupe cyclohexyle ou un groupe cyclohexyloxy dont chacun est éventuellement substitué par un radical alkyle en C₁ à C₆, alkoxy en C₁ à C₆, cyclohexyle ou phényle ; un groupe pyridyloxy portant le cas échéant 1 ou 2 substituants, identiques ou différents, halogéno, alkyle en C₁ à C₄ ou halogénalkyle en C₁ à C₄ ; un groupe phényle, benzyle, phénéthyle, phénoxy, phénylthio, benzyloxy, phénéthyloxy, benzylthio ou styryle portant éventuellement dans chaque cas 1 à 3 substituants, identiques ou différents, alkyle en C₁ à C₁₂, halogéno, cyano, halogénalkyle en C₁ à C₄, alkoxy en C₁ à C₆, halogénalkoxy en C₁ à C₆, (alkoxy en C₁ à C₆)-(alkyle en C₁ à C₆), (alkoxy en C₁ à C₆)-éthylène-oxy, alkylthio en C₁ à C₆, halogénalkylthio en C₁ à C₆ ou triméthylsilyle.

2. Dérivés de diphényloxazoline de formule (I) suivant la revendication 1, dans laquelle
A désigne un groupe phényle qui porte 3 à 5 substituants, identiques ou différents, F, Cl, Br, alkyle en C₁ à C₃, alkoxy en C₁ à C₃, alkylthio en C₁ à C₃, un groupe alkyle en C₁ ou C₂ portant 1 à 5 substituants, identiques ou différents, F et/ou Cl, un groupe alkoxy en C₁ à C₄ portant 1 à 5 substituants, identiques ou différents, F et/ou Cl, un groupe SCF₃, SCHF₂, nitro ou cyano, et
B désigne un groupe phényle qui porte 1 à 5 substituants, identiques ou différents, F, Cl, Br, alkyle en C₁ à C₁₈, (alkoxy en C₁ à C₆)(alkyle en C₁ à C₈), un groupe alkoxy en C₁ à C₈ portant 1 à 6 substituants, identiques ou différents, F et/ou Cl, un groupe alkyle en C₁ ou C₂ portant 1 à 5 substituants, identiques ou différents, F et/ou Cl, un groupe alkoxy en C₁ à C₁₈ et un groupe -(OC₂H₄)₁₋₃-O-(alkyle en C₁ à C₆), un groupe alkylthio en C₁ à C₁₂, un groupe alkylthio en C₁ à C₈ portant 1 à 6 substituants, identiques ou différents, F et/ou Cl, les groupements 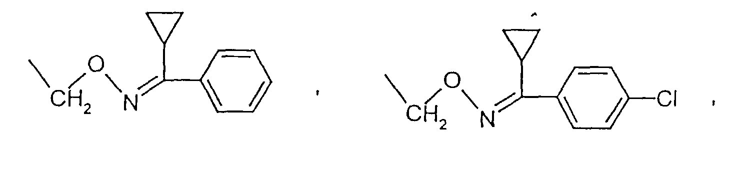 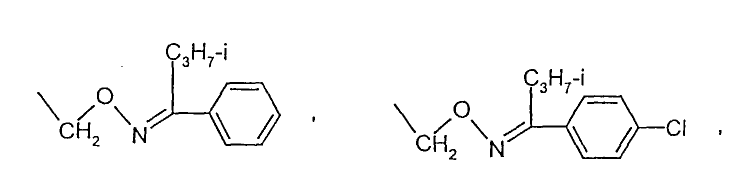 un groupe cyclohexyle ou un groupe cyclohexyloxy portant chacun le cas échéant un substituant alkyle en C₁ à C₄, alkoxy en C₁ à C₄, cyclohexyle ou phényle ; un groupe pyridyloxy portant le cas échéant 1 ou 2 substituants, identiques ou différents, F, Cl ou CF₃ ; 1 un groupe phényle, benzyle, phénéthyle, phénoxy, phénylthio, phénéthyloxy, benzyloxy, benzylthio ou styryle portant le cas échéant 1 à 3 substituants, identiques ou différents, alkyle en C₁ à C₁₂, F, Cl, Br, cyano, CF₃, alkoxy en C₁ à C₄, alkoxy en C₁ à C₄ éventuellement substitué une à six fois identiques ou différentes par F et/ou Cl, (alkoxy en C₁ à C₄)-(alkyle en C₁ à C₄), (alkoxy en C₁ à C₄)-éthylène-oxy, alkylthio en C₁ à C₄, alkylthio en C₁ à C₄ éventuellement substitué une à six fois identiques ou différentes par F et/ou Cl, ou triméthylsilyle.

3. Procédé de production de dérivés de diphényloxazoline de formule (I) suivant la revendication 1, caractérisé en ce que
α) on fait réagir des amino-alcools de formule (II) 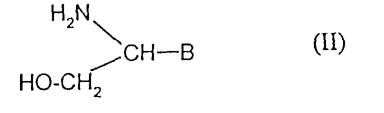 dans laquelle
B a la définition indiquée ci-dessus,
avec un acide carboxylique de formule (III)
A-COOH (III)
dans laquelle
A a la définition indiquée ci-dessus,
avec un agent déshydratant et le cas échéant en présence d'un diluant ;
ou bien
β) on fait réagir des amido-alcools de formule (IV) 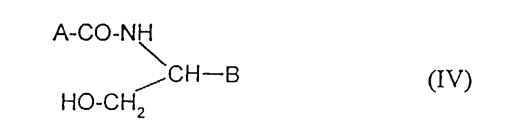 dans laquelle
A et B ont la définition indiquée ci-dessus,
avec un agent déshydratant, éventuellement en présence d'un diluant ;
ou bien
γ) on fait réagir des dérivés d'amides de formule (V) 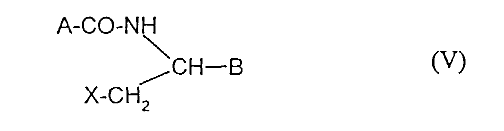 dans laquelle
A et B ont la définition indiquée ci-dessus ; et
X est un groupe partant,
avec une base, éventuellement en présence d'un diluant.

4. Compositions pesticides, caractérisées par une teneur en au moins un composé de formule (I) suivant la revendication 1.

5. Procédé pour combattre des parasites animaux, caractérisé en ce qu'on fait agir des composés de formule (I) suivant la revendication 1 sur des parasites animaux et/ou sur leur milieu.

6. Utilisation de composés de formule (I) suivant la revendication 1, pour combattre des parasites animaux.

7. Procédé de préparation de compositions pesticides, caractérisé en ce qu'on mélange des composés de formule (I) suivant la revendication 1 avec des diluants et/ou des agents tensio-actifs,
